# DEMANDE DE BREVET EUROPEEN

(11) **EP 3 771 474 A1**
(43) Date de publication de la demande: **03.02.2021**
(21) Numéro de dépôt: 20187751.1
(22) Date de dépôt: 24.07.2020
(51) Int. Cl.: A61M 1/36

(54) **MATÉRIAU POUR CAPTURE DE CELLULES CIRCULANTES DANS LE SANG, PROCÉDÉ DE PRÉPARATION ET UTILISATION**

(30) Priorité: 01.08.2019 FR 1908831
(71) Demandeur: I.Ceram, 87280 Limoges (FR)
(72) Inventeur: KERISIT, André, 87000 Limoges (FR); POLI, Evelyne, 87270 Couzeix (FR); BARRIERE, Guislaine, 87400 Saint-Leonard de Noblat (FR); LEVEQUE, Guillaume, 87510 Nieul (FR); RIGAUD, Michel, 87220 Feytiat (FR)
(74) Mandataire: August Debouzy

(57) **Abrégé**

[La présente invention porte sur un matériau choisi parmi la céramique en alumine greffé par au moins un aptamère, au moins un anticorps ou leur combinaison pour la capture des cellules étrangères circulantes, de préférence des cellules tumorales circulantes, la méthode de préparation d'un tel matériau, son utilisation et dispositif le comprenant.

SEQUENCE LISTING

<110> I.CERAM

<120> Matériau pour capture de cellules circulantes dans le sang, procédé de
préparation et utilisation

<130> 5022-2302

<140> EP20187751.1
<141> 2020-07-24

<150> FR1908831
<151> 2019-08-01

<160> 1

<170> BiSSAP 1.3.6

<210> 1
<211> 72
<212> DNA
<213> Homo sapiens


<220>
<223> Synthetic anti-MUC1 aptamer

<400> 1
gggagacaag aataaacgct caagcagttg atcctttgga taccctggtt cgacaggagg     60

ctcacaacag gc                                                         72

## Description

### DOMAINE DE L'INVENTION

Le domaine de l'invention est celui des matériaux permettant la capture d'éléments cellulaires étrangers circulant dans le sang, de préférence des cellules tumorales circulantes. Plus exactement, la présente invention porte sur un matériau céramique en alumine sur lequel est greffé au moins un aptamère et/ou au moins un anticorps permettant la capture d'éléments cellulaires étrangers circulant dans le sang, de préférence des cellules tumorales circulantes. La présente invention porte également sur un procédé de préparation d'un tel matériau, un dispositif comprenant un tel matériau ainsi que l'utilisation du matériau selon l'invention pour la capture d'éléments cellulaires étrangers circulant dans le sang, de préférence des cellules tumorales circulantes.

### ARRIERE-PLAN TECHNIQUE

Le cancer est une maladie résultant de la prolifération excessive et anarchique de cellules anormales ou malignes pouvant toucher n'importe quelle partie de l'organisme. Lorsque ces cellules malignes se multiplient, elles forment une masse désignée par le terme « tumeur ». Il arrive que des cellules se détachent de la tumeur et passent dans la circulation sanguine et/ou lymphatique, se disséminant dans tout l'organisme et formant des tumeurs secondaires également dénommées métastases. Ces cellules sont désignées par cellules tumorales circulantes (CTC). On estime que 95% à 98% des tumeurs solides produisent des CTC.

On estime également qu'à l'échelle mondiale, 70% des décès liés au cancer du sein ainsi que 80% des décès liés au cancer du poumon et 40% des décès liés au cancer de la prostate sont dus à des métastases.

Par ailleurs, l'efficacité du traitement face au cancer dépend principalement de la possibilité de détruire la tumeur. La chirurgie permet de retirer la tumeur primitive. Cependant, la découverte d'une métastase, même unique, signifie que des cellules tumorales circulantes circulent dans le sang ou la lymphe, et peuvent à tout moment se nicher dans un autre organe pour former une autre métastase. En ce cas, la chirurgie ne suffit pas à traiter le cancer et il est nécessaire de passer à des traitements chimiothérapeutiques plus lourds. La présence de cellules tumorales circulantes dans le sang et/ou la lymphe signifie donc qu'il y a un risque pour le patient de développer des métastases dans d'autres organes.

Les cellules tumorales circulantes présentent également un intérêt sur le plan pronostic. Par exemple, dans le cas du cancer du sein, on estime que le seuil de 5 CTC dans un échantillon de 7.5 ml de sang périphérique constitue un bon pronostic pour la patiente. Au-delà de ce seuil, il s'agit d'un mauvais pronostic.

A ce jour, il existe de nombreuses technologies de comptage mais peu de méthodes connues pour capturer et éliminer les cellules tumorales circulantes. On connait par exemple la technique d'identification et de numération des cellules tumorales circulantes commercialisée sous le nom de CELLSEARCH® System, qui est actuellement la seule technique validée par l'agence américaine de sécurité alimentaire et sanitaire FDA (pour « Food and Drug Administration » en anglais). Cette technique utilise des billes immunomagnétiques, c'est-à-dire des nanoparticules magnétiques recouvertes d'anticorps dirigés contre les protéines EpCam, CD45, cytokératine 8 et/ou cytokératine 19, permettant de détecter et de dénombrer avec précision des CTC dans un échantillon de sang de 7.5 ml. Cette technique est limitée à la détection et numération des CTC émanant d'un cancer du sein, du colon ou de la prostate.

On peut également citer l'utilisation de billes ou de nanoparticules sur lesquelles sont fixées des anticorps permettant de fixer des cellules tumorales circulantes pour leur étude et leur caractérisation. De telles billes sont par exemple commercialisées par la société QIAGEN sous les noms *AdnaTest*® *BreastCancerSelect* ou *AdnaTest*® *BreastCancerDetect* ou pour l'enrichissement de CTC de cancer du sein à partir d'un échantillon de sang périphérique d'une patiente. Les billes présentent à leur surface des anticorps spécifiques des ligands GA733-2, MUC-1 et HER-2.

D'autres techniques mettent en œuvre la fixation d'aptamères pour la détection de cellules tumorales circulantes, ces aptamères étant fixés à des puces en oxyde de silice ou en oxyde de graphène, ou des colonnes magnétiques comprenant des particules d'oxyde de fer.

En raison notamment du volume d'échantillon testé qui ne peut dépasser 10 ml, toutes ces techniques sont limitées à des fins diagnostiques ou de suivi de l'évolution d'un cancer chez un patient ayant été opéré et/ou ayant subi une chimiothérapie. Cependant, aucune de ces techniques ne permet de capturer les cellules tumorales circulantes du sang d'un patient pour les éliminer. En effet, ces techniques ne peuvent être utilisées en pratique pour débarrasser le sang d'un patient des cellules tumorales circulantes, et n'ont d'ailleurs pas cet objectif.

Or, la capture et l'élimination de telles cellules de l'organisme pourraient prévenir et/ou réduire le risque de développement de tumeurs, ce qui permettrait d'améliorer grandement les chances de survie d'un patient atteint de cancer.

Un tel objectif nécessite de tenir compte d'un certain nombre de facteurs. Premièrement, les caractéristiques physiologiques, chimiques et moléculaires des cellules tumorales circulantes dépendent d'une part du type de cancer primitif dont elles se sont détachées et d'autre part du stade de progression de la maladie. En effet, les cellules cancéreuses d'un cancer nouvellement formé peuvent présenter des marqueurs différents des cellules issues d'un cancer moins récent. Le système développé se doit donc d'être adaptable. Deuxièmement, la capture des cellules tumorales circulantes dans une optique clinique nécessite de développer un système permettant le traitement du volume total sanguin d'un patient, c'est-à-dire entre 3 et 5L de sang par patient, voire le traitement de plusieurs fois ce volume comme c'est le cas dans le cadre d'une dialyse rénale. Troisièmement, le système de capture se doit d'être parfaitement biocompatible et inerte vis-à-vis des éléments sanguins. Notamment, le matériau permettant la capture des cellules tumorales circulantes ne doit pas réagir chimiquement ou physiquement avec les éléments sanguins, de préférence ne doit pas retenir les éléments sanguins autres que les cellules tumorales circulantes et ne doit pas relarguer d'éléments potentiellement dangereux pour le patient dans la circulation sanguine extracorporelle.

Outre les cellules tumorales circulantes, le sang peut parfois être contaminé par la présence d'éléments cellulaires étrangers, potentiellement pathogènes, tels que les virus, les champignons, les bactéries. Le sang peut également comprendre des éléments chimiques potentiellement toxiques tels que du plomb, de l'arsenic, des pesticides, des insecticides etc.

La demande internationale WO 2017/186626 porte sur un dispositif de filtration sanguine extracorporel permettant de capter des cellules tumorales circulantes présentes dans le sang d'un patient, ainsi qu'une méthode de capture des cellules tumorales circulantes utilisant un tel système. Selon cette technique, le dispositif comprend une ou plusieurs chambres ainsi qu'une hélice sous la forme d'une vis d'Archimède, les parois des chambres ainsi que la vis présentant à leur surface une combinaison de six anticorps et/ou aptamères permettant de capturer les cellules tumorales circulantes présentes dans le sang d'un patient. Cependant, cette demande ne divulgue pas d'exemple concret de matériau permettant la mise en œuvre de cette technique.

Il existe donc un besoin de créer des matériaux permettant de capter et retenir les cellules circulantes tumorales.

### RESUME DE L'INVENTION

Ces objectifs sont atteints grâce au matériau selon la présente invention, son utilisation ainsi qu'un procédé de greffage dudit matériau et son utilisation.

La présente invention porte sur un matériau céramique alumine greffé par au moins un aptamère, au moins un anticorps ou leurs combinaisons.

Avantageusement, l'au moins un aptamère, l'au moins un anticorps ou leurs combinaison est greffé au matériau céramique alumine par une molécule de liaison.

Avantageusement, la molécule de liaison est un trialkoxysilane de formule N⁻ =N⁺=N-L-Si(OR1)(OR2)(OR3) dans laquelle L désigne un radical hydrocarboné divalent linéaire, ramifié ou cyclique comprenant 1 à 20 atomes de carbone, de préférence 1 à 10 atomes de carbone, et R1, R2, R3 sont identiques ou différents et représentent un groupe alkyle linéaire ou ramifié comprenant 1 à 6 atomes de carbone, de préférence 1 à 3 atomes de carbone.

De préférence, la molécule de liaison est le 3-azidopropyltriéthoxysilane.

Avantageusement, le matériau céramique alumine présente au moins une surface en contact avec le sang d'un patient, ladite surface n'étant pas poreuse.

De préférence, le patient est humain.

De préférence, la surface du matériau en contact avec le sang du patient présente une porosité ouverte nulle.

De préférence, la surface du matériau en contact avec le sang du patient est imperméable.

Avantageusement, la céramique alumine est une alumine dense.

Avantageusement, le matériau est greffé par au moins un aptamère.

Avantageusement, l'au moins un aptamère est de l'ADN simple brin.

Avantageusement, l'au moins un anticorps est formé par l'anticorps entier, le fragment Fv, le fragment Fab ou le fragment F(ab')2.

Avantageusement, l'au moins un aptamère ou au moins un anticorps est un aptamère substitué ou un anticorps substitué par au moins un groupe hydrocarboné linéaire, ramifié ou cyclique comprenant une fonction alcyne terminal ayant pour formule -C≡CH et de 3 à 20 atomes de carbone, de préférence de 10 à 15 atomes de carbone, ledit groupe hydrocarboné étant éventuellement substitué par un ou plusieurs hétéroatomes.

Avantageusement, le ou les hétéroatomes peuvent être choisis parmi O, S, N, P ou un halogène, de préférence, parmi O, N, P, de préférence le ou les hétéroatomes sont des atomes d'oxygène.

De préférence, l'au moins un aptamère est substitué à son extrémité 5'.

Avantageusement, l'au moins un aptamère ou l'au moins un anticorps est capable de se lier à l'au moins l'une des protéines choisies parmi mucine 1 (MUC-1), EpCam, HER-2, EGFR, vimentine, E-cadhérine, N-cadhérine, l'OB-cadhérine, le CD44v6, le CD44v8, PSA, cytokératine 8, cytokératine 19, ABC-G2 et leurs combinaisons.

De préférence, l'au moins un aptamère ou l'au moins un anticorps se lie au moins à la protéine mucine 1 (MUC-1).

Avantageusement, l'au moins un aptamère ou au moins un anticorps est lié à au moins une molécule de marquage.

De préférence, l'au moins un aptamère est lié à au moins une molécule de marquage à son extrémité 3'.

Avantageusement, l'au moins une molécule de marquage est un fluorophore.

Avantageusement, le fluorophore est choisi parmi la phycoérythrine (PE), l'isothiocyanate de fluorescéine (FITC), la gamme d'Alexa Fluor™, la protéine fluorescente verte (ou GFP) ou leurs combinaisons.

De préférence, le fluorochrome est l'isothiocyanate de fluorescéine (FITC).

La présente invention a également pour objet un procédé de préparation du matériau selon l'invention comprenant le greffage de la céramique alumine par au moins un aptamère, au moins un anticorps ou leur combinaison.

Avantageusement, l'au moins un aptamère, au moins un anticorps ou leur combinaison est greffé audit matériau céramique alumine par une molécule de liaison, ledit procédé comprenant au moins les étapes suivantes:
- liaison de la molécule de liaison au matériau céramique alumine par réaction d'une ou plusieurs fonctions alkoxy de la molécule de liaison avec un ou plusieurs groupements hydroxyles du matériau céramique alumine ; et
- liaison de l'aptamère ou de l'anticorps à la molécule de liaison par réaction de chimie click.

Avantageusement, l'au moins un aptamère ou l'au moins un anticorps est un aptamère substitué ou un anticorps substitué par au moins un groupe hydrocarboné linéaire, ramifié ou cyclique comprenant une fonction alcyne terminal ayant pour formule -C=CH et de 3 à 20 atomes de carbone, de préférence de 10 à 15 atomes de carbone, ledit groupe hydrocarboné étant éventuellement substitué par un ou plusieurs hétéroatomes.

Avantageusement, l'aptamère ou l'anticorps est substitué et la liaison de l'aptamère substitué ou de l'anticorps substitué à la molécule de liaison est mise en œuvre par réaction de la fonction alcyne terminale de l'aptamère substitué ou de l'anticorps substitué avec la fonction azoture terminale de formule -N=N⁺=N⁻ de la molécule de liaison.

Avantageusement, l'étape de liaison de la molécule de liaison au matériau céramique alumine est mise en œuvre en présence d'un solvant organique.

De préférence, le solvant organique est le toluène.

De préférence, l'étape de liaison de la molécule de liaison au matériau céramique alumine est mise en œuvre dans des conditions anhydres.

Avantageusement, l'étape de liaison de la molécule de liaison au matériau céramique alumine est mise en œuvre sous atmosphère inerte.

Avantageusement, le procédé selon l'invention peut comprendre une étape avant l'étape de liaison de la molécule de liaison au matériau céramique alumine dans laquelle le matériau alumine est préalablement chauffé, de préférence à une température supérieure à 100°C.

Avantageusement, l'étape de liaison de la molécule de liaison au matériau céramique alumine est mise en œuvre à une température allant de 30 à 120°C, de préférence de 50 à 100°C.

Avantageusement, l'étape de liaison de l'aptamère substitué ou de l'anticorps substitué à la molécule de liaison est mise en œuvre en présence d'un ou plusieurs solvants organiques, de préférence l'acétonitrile, ou une amine, de préférence la triméthylamine ou la triéthylamine.

Avantageusement, l'étape de liaison de l'aptamère substitué ou de l'anticorps substitué à la molécule de liaison est mise en œuvre en présence d'un catalyseur, supporté ou non supporté.

Avantageusement, le catalyseur est un catalyseur métallique, de préférence un catalyseur au cuivre.

De préférence, le catalyseur au cuivre est le bromotris(triphénylphosphine) de cuivre I.

Avantageusement, l'étape de liaison de l'aptamère substitué ou de l'anticorps substitué à la molécule de liaison est mise en œuvre à une température allant de 30 à 120°C, typiquement de 50 à 100°C.

Avantageusement, l'étape de liaison de la molécule de liaison au matériau céramique alumine est réalisée avant l'étape de liaison de l'aptamère substitué ou de l'anticorps substitué avec la molécule de liaison.

La présente invention a également pour objet un dispositif de capture des cellules étrangères circulantes comprenant le matériau tel que décrit ci-dessus et au moins un logement.

De préférence, le dispositif de capture est un dispositif de capture de cellules tumorales circulantes.

Avantageusement, le dispositif est un dispositif extracorporel de capture.

Avantageusement, le logement du dispositif selon l'invention présente au moins une entrée pour l'entrée du sang dans le logement et une sortie pour la sortie du sang dans le logement.

Avantageusement, le dispositif selon l'invention présente en outre un outil de capture des cellules étrangères circulantes.

Avantageusement, le logement présente une forme tubulaire.

Avantageusement, l'outil de capture se présente sous la forme d'une hélice ou d'une vis d'Archimède comprenant un axe de rotation.

Avantageusement, l'axe de rotation comprend un aimant à chacune de ses extrémités.

Avantageusement, le logement du dispositif comprend un aimant.

La présente invention a également pour objet le matériau selon l'invention pour son utilisation dans la capture des cellules étrangères circulantes, de préférence des cellules tumorales circulantes.

Avantageusement, le matériau selon l'invention est utilisé pour améliorer l'efficacité d'une chimiothérapie.

Avantageusement, le matériau selon l'invention est utilisé pour réduire le risque de formation de métastase.

La présente invention a également pour objet un aptamère, un anticorps ou leur combinaison greffé sur un matériau céramique alumine selon l'invention pour la capture de cellules étrangères circulantes, de préférence des cellules tumorales circulantes.

Avantageusement, les cellules tumorales circulantes sont issues d'un cancer du sein, d'un cancer du poumon, d'un cancer de la prostate, d'un cancer colorectal, d'un cancer de la vessie, d'un cancer de l'estomac, d'un mélanome.

La présente invention a également pour objet un procédé de capture des cellules étrangères circulantes, de préférence des CTC, mettant en œuvre le matériau selon l'invention.

### DESCRIPTION DETAILLEE DE L'INVENTION

La présente invention porte sur un matériau céramique alumine greffé par au moins un aptamère, au moins un anticorps ou leur combinaison. Au sens de l'invention, le greffage de l'au moins un aptamère, l'au moins un anticorps ou leur combinaison est covalent. De préférence, la céramique d'alumine présente un pourcentage d'alumine supérieur à 95% en poids, de manière davantage préférée supérieure à 99% en poids.

Dans le contexte de l'invention, l'alumine est de formule Al₂O₃. La céramique alumine présente typiquement des groupements hydroxyles à sa surface.

Le matériau céramique alumine selon l'invention présente l'avantage d'être biocompatible et chimiquement inerte vis-à-vis des fluides corporels. Ainsi, contrairement à la silice, la céramique d'alumine ne se dégrade pas avec le temps et ne relarguera donc aucun élément dans le sang du patient. Par ailleurs, la silice ne peut être mise en forme de manière dense comme la céramique.

Selon un mode de réalisation, le matériau selon l'invention peut être greffé par une combinaison d'aptamères, une combinaison d'anticorps ou une combinaison d'aptamères et d'anticorps.

Le matériau selon l'invention présente au moins une surface en contact avec le sang du patient. L'au moins un aptamère, l'au moins un anticorps ou leurs combinaisons sont greffés sur la surface en contact avec les fluides corporels du patient. Bien que la présente invention trouve son application pour le traitement du sang d'un patient, il est entendu qu'elle peut également être utilisé pour traiter tout autre fluide corporel d'un patient. Dans le contexte de la présente invention, on entend par « fluide corporel » tout fluide produit par le corps humain tel que le sang, le sérum, l'urine, la lymphe, le chyle etc. De préférence, le patient est humain.

Dans un mode de réalisation, le matériau selon l'invention présente une surface en contact avec le sang d'un patient, ladite surface n'étant pas poreuse. Dans le contexte de la présente invention, on considère qu'une surface est non poreuse lorsque la surface présente des pores dont la taille est inférieure à 5µm, de préférence inférieure à 2 µm, de manière davantage préférée inférieure à 1 µm. Dans un mode de réalisation particulièrement préféré, la taille des pores de la surface du matériau céramique alumine est nulle. La porosité ouverte de la surface en contact avec le sang du patient est donc nulle. La taille des pores peut être mesurée par microscopie avec analyse de l'image et mesures des « diamètres ». La porosité peut également être mesurée par porosimétrie au mercure. La porosité est définie par le rapport entre le volume occupé par les pores sur le volume total de la céramique, le volume total comprenant la somme du volume des pores et du volume de l'alumine.

Le matériau céramique alumine selon l'invention est donc biocompatible et hémocompatible : il n'entraine aucune réaction d'hémolyse ou de coagulation. Dans le contexte de la présente invention, on considère qu'un matériau est hémocompatible s'il permet la conservation des équilibres osmotiques acidobasiques du sang, s'il n'altère ou ne capture pas les protéines plasmatiques, les éléments figurés du sang, les cellules du tissu vasculaire et s'il n'active pas les systèmes de l'hémostase et du complément. En d'autres termes, la faible porosité de la surface du matériau en contact avec le sang du patient, et de préférence son absence de porosité, permet au matériau selon l'invention d'être utilisé pour le traitement du volume de ce sang en toute innocuité pour le patient.

En effet, selon l'invention, le matériau céramique alumine greffé par au moins un aptamère ou anticorps est destiné à entrer en contact avec le sang du patient pour capturer les cellules étrangères circulantes, de préférence les CTC. Si le matériau selon l'invention était poreux, des éléments sanguins du patient seraient susceptibles d'être retenus dans les pores du matériau. Or, un objectif de la présente invention est de fournir un matériau ne capturant que les cellules étrangères circulantes, de préférence les CTC, dans le sang du patient et non les éléments du sang du patient tel que les hématies, les leucocytes, les plaquettes, les éléments chimiques, sans induire de réaction indésirable telle qu'une hémolyse, une dénaturation des protéines plasmatiques ou une agrégation des plaquettes par exemple. En d'autres termes, la surface du matériau selon l'invention est imperméable au sang, c'est-à-dire qu'elle ne laisse pas traverser le sang.

Une céramique alumine convenant particulièrement à la mise en œuvre de l'invention peut être une céramique dense. Dans le contexte de la présente invention, on considère qu'un matériau céramique en alumine est dense si sa densité est supérieure à 3,5 g/cm³, de préférence au moins 3,8 g/cm³. La densité est mesurée en divisant la masse du matériau par son volume. De préférence, le matériau céramique respecte la norme DIN VDE 0335.

La présente invention fournit donc un matériau, biocompatible et inerte chimiquement vis-à-vis des fluides corporels du patient et notamment du sang. Le greffage du matériau céramique alumine selon l'invention par au moins un aptamère ou au moins un anticorps permet de capturer les éléments indésirables circulant dans le sang d'un patient. Dans le contexte de la présente invention, on entend par capturer le fait que la cellule étrangère se lie à l'aptamère et/ou l'anticorps de manière suffisamment forte pour qu'elle ne se redétache pas pour retourner dans le sang du patient. En particulier, lorsque l'au moins un aptamère ou anticorps est spécifique d'un marqueur de CTC, le matériau selon l'invention permet de capter les CTC circulant dans le sang du patient et de l'en débarrasser. L'utilisation du matériau selon l'invention peut donc être utilisée à des fins thérapeutiques pour éliminer les CTC, ou à tout le moins réduire le nombre de CTC dans le sang d'un patient afin de réduire le risque de formation de métastases. Le matériau selon l'invention permet en outre d'améliorer l'efficacité des chimiothérapies. En effet, en réduisant le nombre de CTC dans le sang du patient, le matériau selon l'invention permet que les substances chimiothérapeutiques soient plus efficaces sans pour autant augmenter leur dosage.

Le matériau selon l'invention peut également être greffé par plusieurs aptamères ou anticorps différents, ou une combinaison d'anticorps et d'aptamères. Les aptamères ou anticorps peuvent être soit :
- dirigés contre différentes parties de la même molécule cible,
- être dirigés contre des molécules différentes, ou
- une combinaison de ces deux options c'est-à-dire que le matériau selon l'invention peut comprendre plusieurs aptamères ou anticorps dont certains seront dirigés contre une même cible tandis que les autres aptamères ou anticorps seront dirigés contre une autre cible.

De préférence, l'aptamère ou l'anticorps est fait à façon pour chaque patient. En d'autres termes, l'aptamère ou l'anticorps peut être conçu pour réagir spécifiquement avec les marqueurs exprimés par les cellules circulant dans le sang du patient.

Les aptamères et/ou les anticorps peuvent être répartis de manière uniforme à la surface du matériau ou de manière hétérogène. De préférence, les aptamères ou anticorps sont répartis de manière homogène à la surface de la céramique de sorte à capturer et éliminer efficacement les éléments étrangers du sang. La concentration des aptamères et/ou anticorps greffés à la surface du matériau peut être comprise entre 0,01 nmol/cm² et 10 nmol/cm², de préférence entre 0,1 nmol/cm² et 1 nmol/cm², de manière davantage préférée entre 0,3 nmol/cm² et 0,5 nmol/cm².

Le matériau selon l'invention peut également être greffé par au moins un agent anticoagulant pour éviter que le sang du patient ne coagule pendant la mise en œuvre du matériau pour la capture et l'élimination des CTC. De préférence, l'au moins un agent anticoagulant peut être choisi parmi l'héparine, un agent anti-vitamine K, un inhibiteur direct du facteur Xa, un inhibiteur direct de la thrombine ou leurs combinaisons.

L'agent anti-vitamine K peut être choisi parmi les dérivés coumariniques, les dérivés de l'indanedione ou leurs combinaisons. Les dérivés coumariniques peuvent être l'acénocoumarol et la warfarine. Les dérivés de l'indanedione peuvent être le fluindione.

Tout agent anticoagulant autorisé par les autorités de santé convient à la mise en œuvre de la présente invention.

### Aptamère

Selon un mode de réalisation préféré de l'invention, le matériau céramique est greffé par un ou plusieurs aptamères. De préférence, l'un ou plusieurs aptamères est greffé de manière covalente à la surface du matériau.

Dans le contexte de la présente invention, un aptamère est un oligonucléotide synthétique permettant de fixer un ligand spécifique. On entend par « oligonucléotide » une séquence d'ADN ou d'ARN de cent paires de bases ou moins. Leur sélectivité et leurs propriétés de fixation sont comparables à celles des anticorps. L'oligonucléotide peut être de l'acide ribonucléique (ARN) ou de l'acide désoxyribonucléique (ADN) simple brin. De préférence, l'aptamère est de l'ADN simple brin.

L'aptamère est fait à façon afin d'être spécifique des marqueurs exprimés par les cellules tumorales. L'homme du métier connait les méthodes de synthèse et de sélection des aptamères. Parmi ces méthodes, il convient de citer la méthode SELEX (pour « Systematic Evolution of Ligands by EXponential enrichment » en anglais qui peut se traduire par « évolution systématique de ligands par enrichissement exponentiel »). Brièvement, cette méthode de sélection est une méthode itérative, basée sur la répétition des étapes jusqu'à obtenir l'aptamère désiré. Elle nécessite une banque d'oligonucléotides dont les extrémités 5' et 3' sont connues et dont la séquence centrale est aléatoire. Le caractère aléatoire de la séquence centrale est obtenu en faisant réagir les quatre bases de l'ADN ou de l'ARN lors de l'étape d'élongation. Les quatre bases de l'ADN sont adénine, cytosine, guanine et thymine et les quatre bases de l'ARN sont adénine, cytosine, guanine et uracile. Cela permet d'obtenir des oligonucléotides de longueur différente et de séquence différente. L'étape suivante consiste à sélectionner les oligonucléotides qui se lient avec la meilleure affinité possible à la molécule cible. Plusieurs techniques connues de l'homme du métier permettent la séparation des oligonucléotides qui ont reconnu la molécule cible des autres oligonucléotides qui ne reconnaissent pas la cible. On peut citer à titre d'exemple de méthode de séparation la chromatographie d'affinité en gel natif, la séparation sur membrane, ou l'utilisation de billes magnétiques. Les oligonucléotides sont ensuite séparés de la molécule d'intérêt et sont amplifiés par clonage ou par PCR. Les étapes de sélection des oligonucléotides et d'amplification sont répétées plusieurs fois afin d'obtenir des oligonucléotides ayant la haute affinité pour la molécule cible. Les oligonucléotides sélectionnés sont appelés des aptamères. La technique SELEX fournit de bons résultats et des oligonucléotides avec une constante de dissociation très faible.

L'aptamère a des propriétés de sélectivité et d'affinité comparables à celles des anticorps. Les propriétés de spécificité et d'affinité des aptamères permettent leur utilisation afin de capter les cellules étrangères circulantes, de préférence les CTC, dans le sang d'un patient et les lier de manière suffisamment forte pour qu'une cellule circulante ne se détache pas de l'aptamère auquel elle est liée et ne retourne pas dans le sang du patient. Ainsi, le matériau selon l'invention permet de capter et retirer les cellules étrangères circulantes, de préférence les CTC du sang d'un patient.

### Anticorps

Selon un mode de réalisation, le matériau selon l'invention peut être greffé par un ou plusieurs anticorps. L'au moins un anticorps est formé par un anticorps entier, un fragment Fv, un fragment Fab ou un fragment F(ab')2. De préférence, l'un ou plusieurs anticorps est greffé de manière covalente à la surface du matériau.

Le terme "anticorps", tel qu'utilisé ici, désigne les molécules d'immunoglobulines constituées de quatre chaînes polypeptidiques, deux chaînes lourdes (H) et deux chaînes légères (L) interconnectées par des liaisons disulfure. Chaque chaîne lourde est composée d'une région variable de chaîne lourde (VH) et d'une région constante de chaîne lourde. La région constante de la chaîne lourde est composée de trois domaines, CH1, CH2 et CH3. Chaque chaîne légère est composée d'une région variable de chaîne légère (VL) et d'une région constante de chaîne légère. La région constante de la chaîne légère est composée d'un domaine CL. Les régions VH et VL peuvent être subdivisées en régions d'hypervariabilité, appelées régions déterminant la complémentarité (CDR), entrecoupées de régions plus conservées, appelées régions cadres (FR). Chaque VH et VL est composé de trois CDR et de quatre FR, disposés en amino-terminus à carboxy-terminus dans l'ordre suivant : FR1, CDR1, CDR1, FR2, CDR2, FR3, CDR3, FR4.

Les portions d'anticorps, comme les fragments Fv, Fab et F(ab')2, peuvent être préparées à partir d'anticorps entiers en utilisant des techniques conventionnelles, comme la digestion par papaïne ou pepsine d'anticorps entiers. De plus, les anticorps ou les portions d'anticorps Fv, Fab et F(ab')2 peuvent être obtenus en utilisant les techniques conventionnelles d'ADN recombinant. On entend par « fragment Fv » le plus petit fragment constitué par les chaines lourdes et légères de la région variable et conservant les proproétés de liaison de l'anticorps. On entend par « fragment Fab » le fragment monovalent d'anticorps formé par la chaine légère entière (partie variable et partie constante) et d'une partie de la chaine lourde. On entend par « fragment F(ab')2 » l'association de deux fragments Fab reliés par la région charnière (en anglais « *hinge* ») de l'anticorps.

Le terme " anticorps isolé ", tel qu'utilisé ici, désigne un anticorps qui est essentiellement exempt d'autres anticorps ayant des spécificités antigéniques différentes (par exemple, un anticorps isolé qui se lie spécifiquement à MUC-1 est essentiellement exempt d'anticorps qui se lient spécifiquement à des antigènes autres que MUC-1). De plus, un anticorps isolé peut être pratiquement exempt d'autres substances cellulaires et/ou chimiques.

Les anticorps convenant à la mise en œuvre de l'invention peuvent être des anticorps polyclonaux ou monoclonaux, produits selon toute méthode bien connue de l'homme de l'art. De préférence, l'au moins un anticorps est un anticorps monoclonal.

L'anticorps pour la mise en œuvre de l'invention est de préférence humanisé.

L'anticorps peut en outre être dirigé vers un marqueur spécifique d'une cellule étrangère à l'organisme afin de capter les cellules étrangères circulant dans le sang et de retirer ces cellules étrangères circulantes.

De préférence, l'anticorps ou le fragment d'anticorps Fv, Fab ou F(ab')2 est greffé au matériau selon l'invention par l'intermédiaire d'une molécule de liaison.

### Substitution

De préférence, l'aptamère ou l'anticorps est substitué par au moins un groupe hydrocarboné linéaire, ramifié ou cyclique comprenant une fonction alcyne terminale ayant pour formule -C=CH et de 3 à 20 atomes de carbone, de préférence de 10 à 15 atomes de carbone, ledit groupe hydrocarboné pouvant comprendre au moins un hétéroatome. La présence de la fonction alcyne terminale permet de faire réagir l'aptamère ou l'anticorps avec la molécule de liaison de manière covalente.

Le ou les hétéroatomes peuvent être choisis parmi O, P, N, S et un halogène, de préférence, parmi O, N, P, de manière davantage préférée le ou les hétéroatomes sont des atomes d'oxygène.

Au sens de la présente invention, un «groupe hydrocarboné» est un groupement comprenant des atomes de carbone et d'hydrogène, linéaire, ramifié ou cyclique, substitué ou non substitué par un ou plusieurs hétéroatomes. Le groupe hydrocarboné peut être saturé ou insaturé.

Selon un mode de réalisation, l'aptamère ou l'anticorps est substitué par un groupe hydrocarboné linéaire ou ramifiée comprenant une fonction alcyne terminale et comprenant de 3 à 20 atomes de carbone, ladite chaîne hydrocarbonée étant substituée par au moins une fonction choisie parmi les fonctions hydroxyle et carbonyle. De préférence, la ou les ramifications du groupe hydrocarboné lorsqu'elles sont présentes sur le groupe hydrocarboné, comprennent de 1 à 5 atomes de carbone, de préférence de 1 à 3 atomes de carbone, avantageusement 1 à 2 atomes de carbone.

Selon un mode de réalisation, l'aptamère ou l'anticorps est substitué par un groupe alkyle linéaire ou ramifié comprenant une fonction alcyne terminale et au moins une fonction carbonyle et au moins une fonction hydroxyle, ledit groupe alkyle comprenant de 10 à 15 atomes de carbone. De préférence, la ou les ramifications du groupe alkyle lorsqu'elles sont présentes sur le groupe alkyle, comprennent de 1 à 5 atomes de carbone, de préférence de 1 à 3 atomes de carbone, avantageusement 1 à 2 atomes de carbone.

L'aptamère substitué ou l'anticorps substitué peut être disponible commercialement ou synthétisé selon des méthodes bien connues de l'homme du métier.

Ainsi, le greffage de l'aptamère ou de l'anticorps au matériau est suffisamment résistant pour éviter son détachement du matériau et que l'aptamère ou l'anticorps ne se retrouve dans le sang périphérique du patient.

### Cellules étrangères circulantes

Le matériau selon l'invention permet de capter tout élément circulant dans le sang. Le matériau selon l'invention permet notamment de capter des éléments cellulaires étrangers à l'organisme et se trouvant dans le sang.

Notamment, les cellules étrangères peuvent être un virus, une bactérie, un champignon, une cellule tumorale circulante, une cellule animale étrangère à l'organisme ou leurs combinaisons.

Selon un mode de réalisation préféré, les cellules étrangères circulantes sont des cellules tumorales circulantes. En effet, les cellules tumorales se distinguent souvent des cellules « normales » du tissu concerné car elles présentent des marqueurs cellulaires différents.

### Marqueurs

L'au moins un aptamère ou l'au moins un anticorps sont de préférence dirigés vers des marqueurs des cellules étrangères circulantes.

Dans un mode de réalisation, l'aptamère ou l'anticorps peut être dirigé contre une protéine de la capside d'un virus ou contre une protéine, un sucre ou une glycoprotéine de la capside d'un virus afin de piéger le virus. A titre d'exemple, l'aptamère ou l'anticorps peut être dirigés contre l'hémagglutinine.

Dans le contexte de la présente invention, le terme "sucre" désigne les monosaccharides, les disaccharides et les polysaccharides. Les sucres comprennent, entre autres, le saccharose, le glucose, le dextrose et d'autres sucres, sans toutefois s'y limiter.

Dans le contexte de la présente invention, une protéine désigne une macromolécule biologique, formée d'une ou de plusieurs chaînes polypeptidiques. Chacune de ces chaînes est constituée de l'enchaînement de résidus d'acides aminés liés entre eux par des liaisons peptidiques. Dans le contexte de la présente invention, un acide aminé est un acide carboxylique qui possède également un groupe fonctionnel amine. De tels composés organiques ont donc à la fois un groupe carboxyle -COOH et un groupe amine, par exemple une amine primaire - NH2 ou une amine secondaire -NH-. Dans le contexte de la présente invention, Une glycoprotéine est une protéine portant un ou plusieurs groupements oligosides, un oligoside étant un oligomère formé d'un nombre de monosaccharides, identiques ou différents, par liaison glycosidique alpha ou beta.

L'aptamère ou l'anticorps peut également être dirigé contre une molécule présente à la surface d'une bactérie. A titre d'exemple de marqueur bactérien, on peut citer les lipopolysaccharides (LPS) présents dans la membrane externe des bactéries Gram négatif.

Concernant les cellules tumorales circulantes (CTC), il existe différents marqueurs en fonction du type de cancer dont le patient est atteint. La littérature scientifique fournit à l'homme du métier les marqueurs spécifiques qu'il doit rechercher en fonction d'un type de cancer précis. Parmi les marqueurs auxquels aptamères peuvent se lier, on peut citer les marqueurs EpCam (pour « EPithelial Cell Adhésion Molecule » en anglais ou molécule d'adhésion des cellules épithéliales), HER-2 (pour « Human Epidermal growth factor Receptor 2 » en anglais ou récepteur 2 du facteur de croissance épidermique humain), EGFR (pour « Epithelial Growth Factor Receptor» en anglais ou récepteur au facteur de croissance épithélial), la protéine mucine-1 (MUC-1), la vimentine, l'E-cadhérine, la N-cadhérine, l'OB-cadhérine, le CD44v6, le CD44v8, le PSA (pour « Prostate Specific Antigen » ou antigène spécifique de la prostate), la cytokératine 8, la cytokératine 19, l'ABC-G2 et leurs combinaisons.

Le marqueur CD44v6 est exprimé par les cellules du cancer du côlon. Les marqueurs HER2 et CD44v8 sont spécifiquement exprimés par les cellules du cancer du sein. Les marqueurs EpCam, EGFR et E-cadhérine sont spécifiques des cellules épithéliales et se trouvent exprimés à la surface de nombreuses cellules issues de carcinomes dont le cancer du poumon non à petites cellules par exemple.

De préférence, l'aptamère ou l'anticorps se lie à la protéine mucine 1. La protéine mucine 1 est une glycoprotéine codée par le gène MUC1 chez l'humain, exprimée au pôle apical de nombreuses cellules épithéliales.

Cette protéine est particulièrement surexprimée par les cellules tumorales circulantes issues d'un cancer du sein, d'un cancer du poumon, d'un cancer de la prostate, d'un cancer colorectal, d'un cancer de la vessie, d'un cancer de l'estomac, d'un mélanome.

Plus précisément, les cellules tumorales circulantes expriment une version de MUC-1 présentant une glycosylation réduite et anarchique, ce qui la rend particulièrement antigénique et la différencie bien de la protéine MUC-1 exprimée par les cellules normales. La protéine MUC-1 est donc un bon marqueur discriminant des cellules tumorales circulantes.

De préférence, l'aptamère anti-mucine 1 est un oligonucléotide d'ADN simple brin de 72 bases ayant la séquence suivante :

5'GGGAGACAAGAATAAACGCTCAAGCAGTTGATCCTTTGGATACCCTGGTT
CGACAGGAGGCTCACAACAGGC3'

Le matériau selon l'invention greffé par l'aptamère ou l'anticorps se liant à MUC-1 permet donc de capter les CTC exprimant ce marqueur et d'éliminer un nombre conséquent de CTC de différents cancers.

Dans un mode de réalisation particulièrement avantageux, le matériau selon l'invention est greffé par plusieurs aptamères et/ou anticorps permettant de reconnaitre plusieurs marqueurs différents et/ou différentes parties d'un marqueur.

### Molécule de liaison

L'au moins un aptamère et/ou l'au moins un anticorps peut être greffé audit matériau par une molécule de liaison. La molécule de liaison permet de lier de manière covalente l'aptamère et/ou l'anticorps aux fonctions hydroxyles accessibles présentes à la surface de la céramique d'alumine. La molécule de liaison agit en outre comme un espaceur entre l'aptamère ou l'anticorps et le matériau biocompatible. En effet, un aptamère ou un anticorps est une molécule volumineuse. La liaison directe de l'aptamère ou de l'anticorps sur le matériau créerait un encombrement stérique qui rendrait l'aptamère ou l'anticorps peu disponible, voire indisponible, pour se lier aux éléments cellulaires circulants.

Selon un mode de réalisation, l'aptamère est lié à la molécule de liaison au niveau de son extrémité 5'.

La molécule de liaison comprend de préférence une fonction réactive capable de réagir avec l'aptamère substitué ou l'anticorps substitué par une réaction de chimie-click. Ainsi, la molécule de liaison comprend de préférence une fonction azoture terminale -N=N⁺=N⁻ permettant de réagir avec l'alcyne terminal de l'aptamère substitué ou de l'anticorps substitué pour former un cycle de type triazole.

De préférence, la molécule de liaison est un trialkoxysilane de formule N⁻ =N⁺=N-L-Si(OR1)(OR2)(OR3) dans laquelle L désigne un groupe hydrocarboné divalent linéaire, ramifié ou cyclique comprenant 1 à 20 atomes de carbone, de préférence 1 à 10 atomes de carbone, et R1, R2, R3 sont identiques ou différents et représentent un groupe alkyle linéaire ou ramifié comprenant 1 à 6 atomes de carbone, de préférence 1 à 3 atomes de carbone.

Dans le contexte de l'invention, un groupe alkyle désigne une chaîne hydrocarbonée saturée monovalente.

Dans le contexte de l'invention, un groupe alkoxy de formule -(OR) désigne un groupe alkyle lié à un atome d'oxygène.

Selon un mode de réalisation, L est un groupe hydrocarboné divalent, linéaire ou ramifié comprenant de 1 à 10 atomes de carbone. De préférence, L est un groupe alkyle divalent, linéaire ou ramifié comprenant de 1 à 10 atomes de carbone. De préférence encore, L est un groupe alkyle linéaire comprenant de 1 à 6 atomes de carbone.

De préférence, la molécule de liaison est le 3-azidopropyltriéthoxysilane (AzPTES).

La molécule de liaison peut être disponible commercialement ou synthétisée selon des méthodes bien connues de l'homme du métier.

### Marquage de l'aptamère ou de l'anticorps

Pour la mise en œuvre de la présente invention, l'aptamère ou l'anticorps peut également être conjugué à un ou plusieurs marqueur(s) coloré ou de fluorescence. De préférence, l'aptamère est marqué au niveau de son extrémité 3'. Le marquage de l'aptamère ou de l'anticorps peut se faire selon toute méthode bien connue de l'homme du métier. Le marquage de l'aptamère peut notamment se faire par une réaction de chimie click entre le marqueur et l'aptamère.

De préférence, l'aptamère ou l'anticorps est marqué en utilisant une méthode « froide » de marquage. Dans le contexte de la présente invention, une méthode froide de marquage est une méthode de marquage ne faisant pas intervenir de radioactivité. Ainsi, le matériau selon l'invention est plus simple et plus sûr à produire et utiliser. La présence d'un marqueur permet notamment de vérifier que le greffage des aptamères s'est bien déroulé lors de la production du matériau selon l'invention.

L'aptamère ou l'anticorps peut être marqué par un ou plusieurs fluorophore(s) ou par un marqueur produisant une coloration tel que le système biotine/streptavidine, ou leur combinaison. On entend par « fluorophore » un composé chimique pouvant émettre de la lumière après excitation à une longueur d'onde donné. Chaque fluorophore est excitable à une longueur d'onde spécifique et émet de la lumière à une longueur d'onde spécifique. L'homme du métier sait choisir le ou les fluorophore(s) convenant à la mise en œuvre de l'invention. Les fluorophores convenant à la mise en œuvre de la présente invention peuvent être choisis parmi les molécules citées dans le tableau 1 ci-dessous :

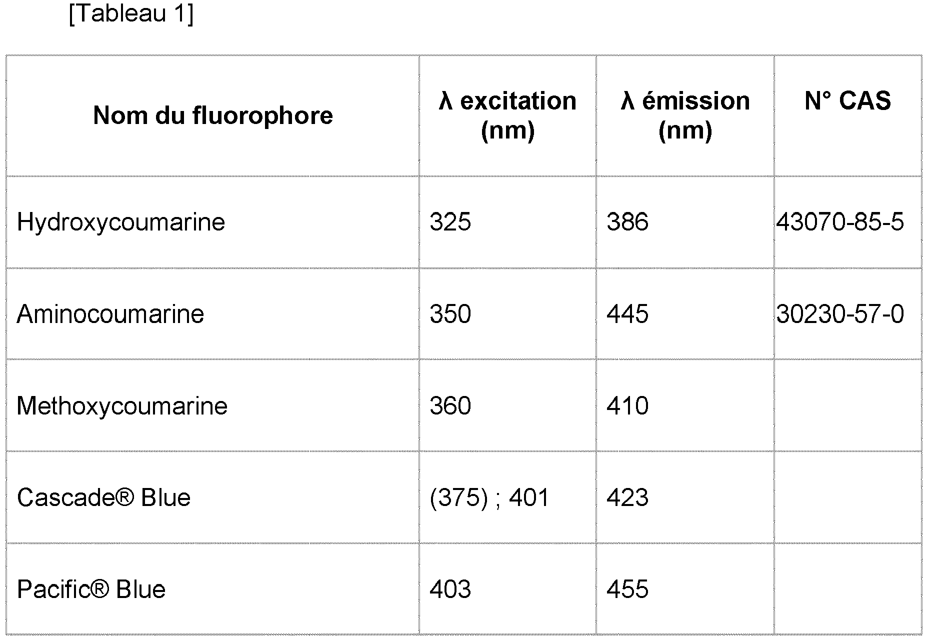

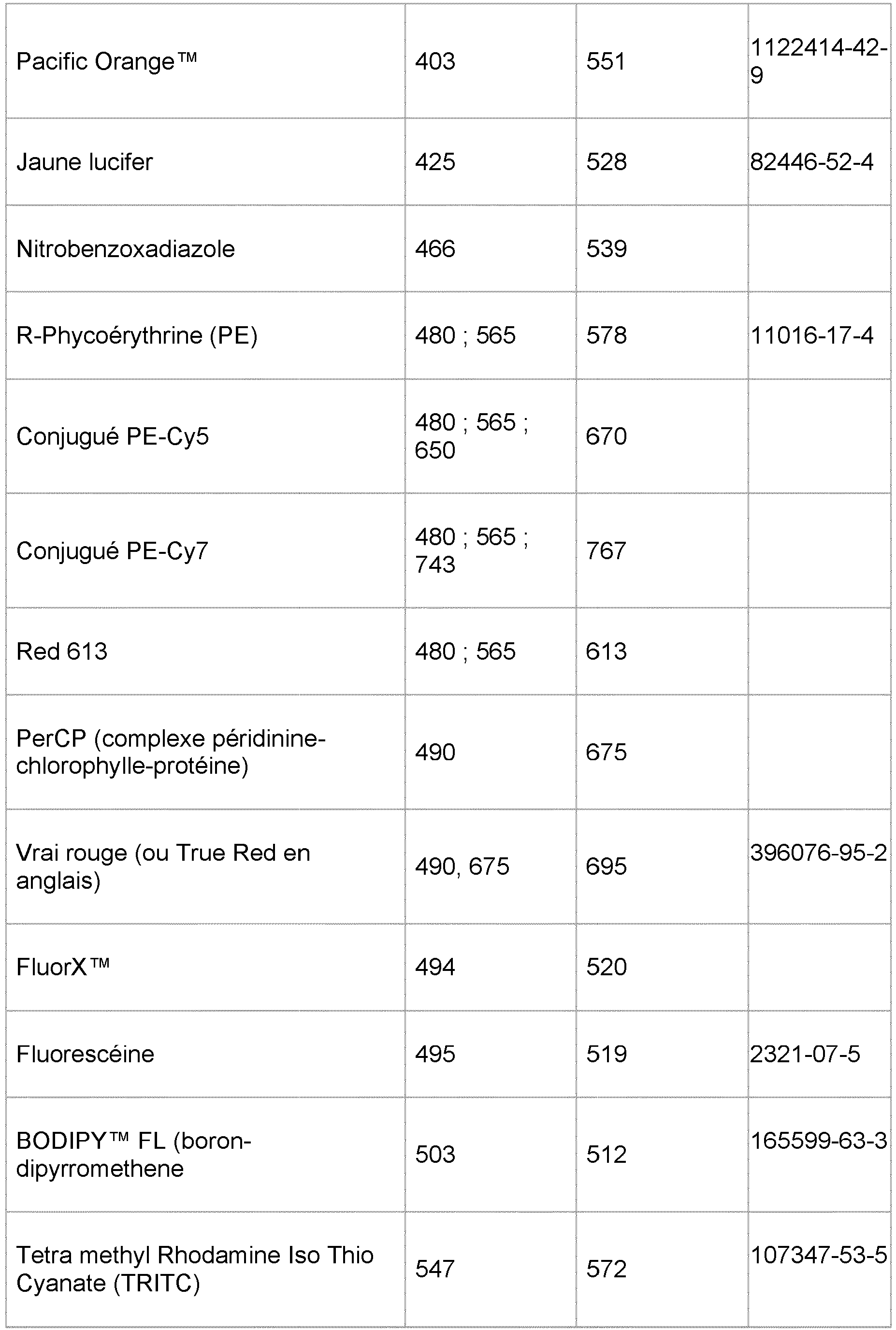

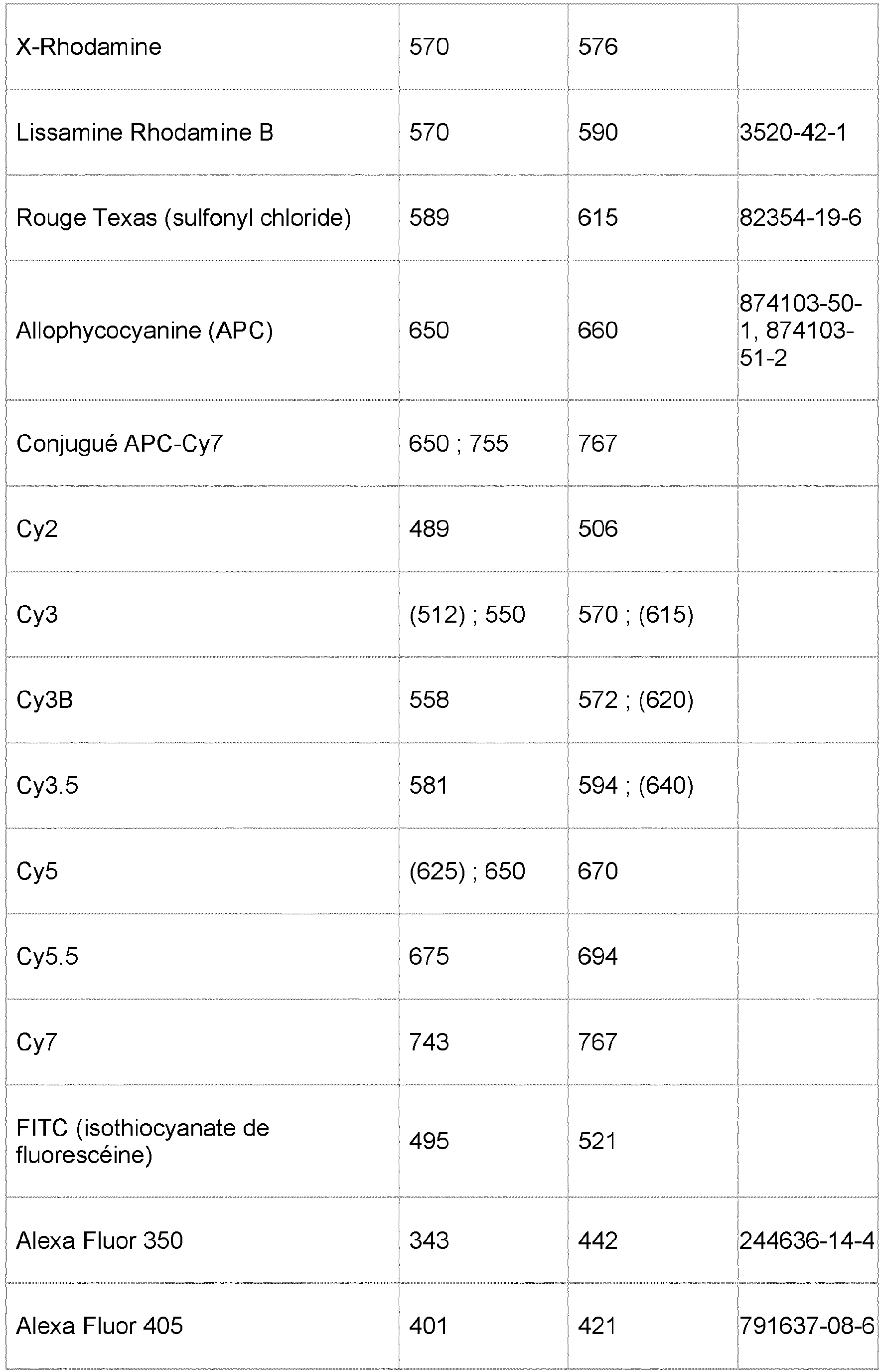

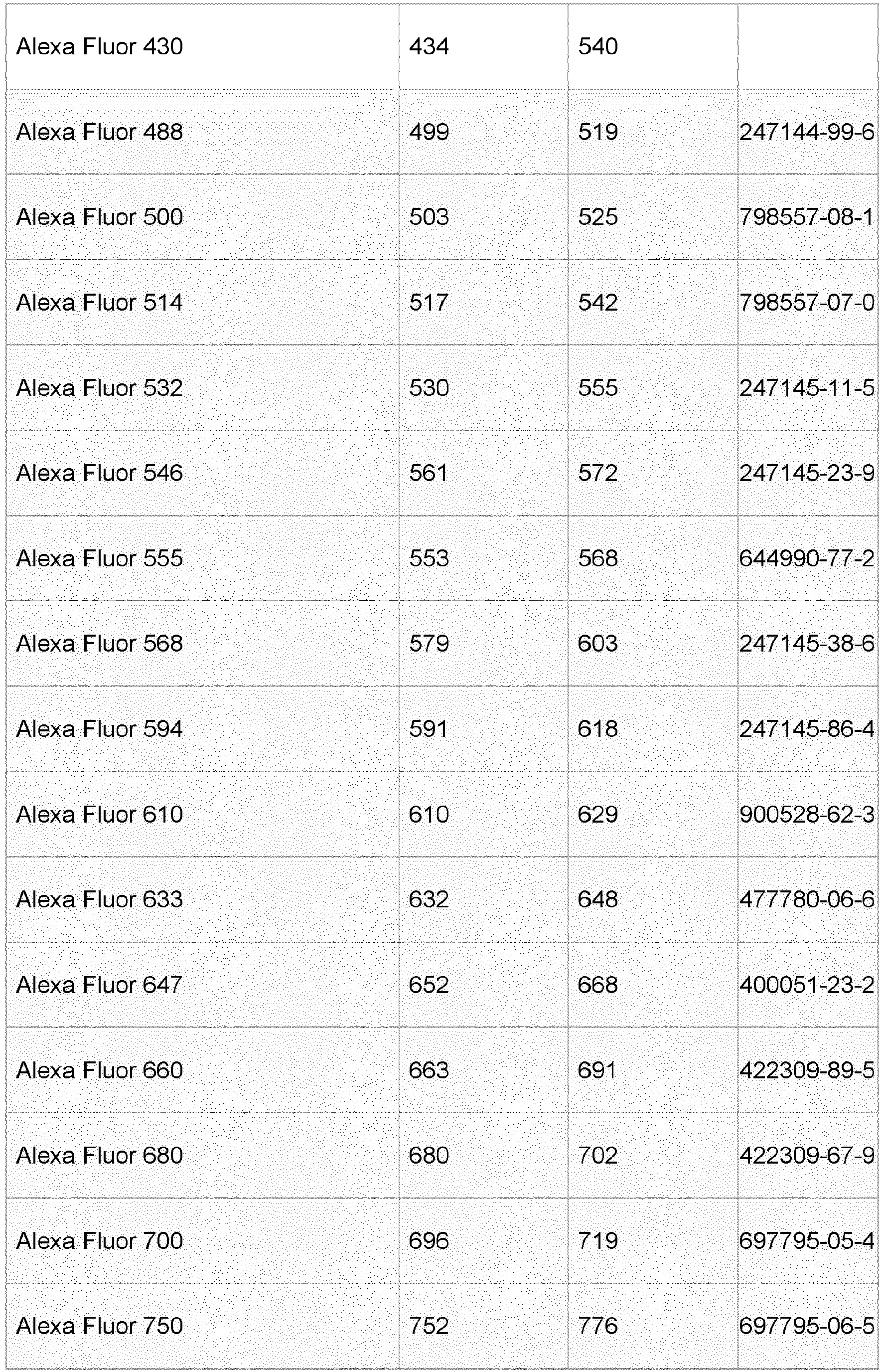

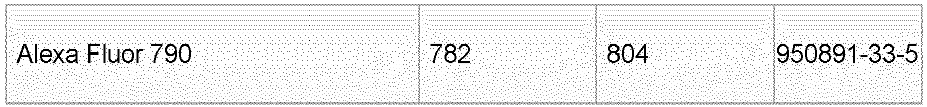

De préférence, le fluorophore est choisi parmi la phycoérythrine (PE), l'isothiocyanate de fluorescéine (FITC), la gamme d'Alexa Fluor™, la protéine fluorescente verte (ou GFP pour « green fluorescent protein » en anglais). De manière davantage préféré, le fluorochrome est l'isothiocyanate de fluorescéine (FITC pour « fluorescein isothiocyanate » en anglais).

Le marquage de l'aptamère ou de l'anticorps peut permettre de vérifier la bonne qualité du greffage de l'aptamère à la surface du matériau. Le marquage de l'aptamère ou de l'anticorps peut également permettre de signaler la liaison de l'aptamère ou de l'anticorps au marqueur cellulaire de la cellule cible. Par exemple, lorsque le matériau comporte une combinaison d'anticorps et/ou d'aptamères, les anticorps et/ou aptamères peuvent être marqués par des couleurs ou des fluorophores différents. Le marquage peut ensuite être ôté selon toute méthode bien connue de l'homme du métier afin de libérer l'aptamère.

### Utilisation

Un autre objet de l'invention est l'utilisation du matériau greffé par au moins un aptamère et/ou au moins un anticorps tel que décrit ci-dessus pour la capture de cellules étrangères circulantes dans le sang d'un patient, et de préférence des CTC. Les CTC sont de préférence issues du sang d'un patient. De préférence, le patient est humain.

Selon cet aspect de l'invention, les CTC sont issues d'un cancer du sein, d'un cancer du poumon, d'un cancer de la prostate, d'un cancer colorectal, d'un cancer de la vessie, d'un cancer de l'estomac ou d'un mélanome. De préférence, les CTC sont issues d'un cancer du sein, de préférence un adénocarcinome du sein. Le matériau selon l'invention peut également être utilisé pour améliorer l'efficacité d'une chimiothérapie et/ou pour réduire le risque de formation de métastase.

En effet, la capture des CTC du sang du patient permet de réduire, voire d'éliminer, la quantité de cellules tumorales circulantes du sang du patient, et donc de réduire les risques de formation d'une métastase. Par ailleurs, le nombre de CTC dans un échantillon de sang est un indice pronostic pour le patient : réduire ce nombre de cellules par volume d'échantillon de sang permet donc d'améliorer le pronostic du patient. Par ailleurs, la réduction de la quantité de CTC dans le sang permet aux substances chimiothérapeutiques d'être plus efficaces, sans pour autant augmenter la dose de substance à injecter au patient.

### Procédé de préparation d'un matériau selon l'invention

La présente invention a également pour objet un procédé de préparation du matériau céramique alumine selon l'invention par greffage de l'au moins un aptamère, de l'au moins un anticorps ou de leur combinaison sur le matériau céramique alumine.

Selon un mode de réalisation, le procédé comprend au moins les étapes suivantes :
- liaison de la molécule de liaison au matériau céramique alumine par réaction d'une ou plusieurs fonctions alkoxy de la molécule de liaison avec un ou plusieurs groupements hydroxyles du matériau céramique alumine ; et
- liaison de l'aptamère substitué ou de l'anticorps substitué à la molécule de liaison par réaction de chimie click.

Ainsi, le matériau selon l'invention est d'abord greffé par la molécule de liaison, puis l'au moins un aptamère substitué ou l'au moins un anticorps substitué est lié à la molécule de liaison. L'étape de liaison de la molécule de liaison au matériau céramique alumine est donc réalisée avant l'étape de liaison de l'aptamère substitué ou de l'anticorps substitué avec la molécule de liaison.

Cela permet notamment de produire le matériau en conditions douces et d'obtenir de bons rendements de greffage de l'au moins un aptamère et/ou anticorps sur le matériau. Par ailleurs, cette variante évite tout risque de liaison directe et indésirable de l'aptamère ou de l'anticorps au matériau céramique. Comme expliqué ci-dessus, il est souhaitable que l'aptamère ou l'anticorps soit lié au matériau céramique via la molécule de liaison pour éviter une saturation trop rapide du matériau par l'encombrement stérique lié aux cellules étrangères, de préférence des cellules tumorales circulantes, fixées sur les aptamères.

Dans le contexte de la présente invention, la condition douce correspond à des conditions de synthèses compatibles avec l'utilisation d'une biomolécule, par exemple en utilisant un solvant adapté tel que l'acétonitrile pour la garder intègre avec un temps réactionnel compris entre 4h et 8h, de préférence 6h et à une température faible comprise entre 30°C-120°C, de préférence environ 90°C.

Dans le contexte de la présente invention, l'expression « chimie click » désigne une classe de réactions chimiques « biocompatibles » utilisées pour joindre à un substrat choisi une biomolécule spécifique (voir https://fr.wikipedia.org/wiki/Chimie click). Typiquement, les réactions click se produisent en synthèse monotope c'est-à-dire une synthèse chimique dans laquelle un réactif subit plusieurs réactions successives et/ou simultanées dans un seul mélange réactionnel.

Selon un mode de réalisation, l'au moins un aptamère et/ou l'au moins un anticorps est substitué et le greffage de l'au moins un aptamère et/ou de l'au moins un anticorps sur le matériau céramique alumine est effectué via une molécule de liaison.

Dans un mode de réalisation de l'invention, la liaison de l'au moins un aptamère substitué ou de l'au moins un anticorps substitué à la molécule de liaison par réaction de la fonction alcyne terminale de l'aptamère substitué avec la fonction azoture terminale de formule -N=N⁺=N⁻ de la molécule de liaison. Cette liaison peut se faire par une cycloaddition alcyne-azoture également appelée cycloaddition de Huisgen 1,3 dipolaire. Typiquement, la réaction de la fonction alcyne terminale de l'aptamère substitué ou de l'anticorps substitué avec la fonction azoture terminale de formule -N=N⁺=N⁻ de la molécule de liaison permet de former un cycle de type 1,2,3-triazole.

Selon un mode de réalisation, l'étape de liaison de la molécule de liaison au matériau céramique alumine est mise en œuvre en présence d'un solvant organique, tel que le toluène, de préférence dans des conditions anhydres, et avantageusement sous atmosphère inerte, typiquement sous argon. Avant cette étape de liaison, le matériau alumine peut être préalablement chauffé par exemple à une température supérieure à 100°C, afin de le sécher et d'éliminer l'humidité.

Selon un mode de réalisation, l'étape de liaison de la molécule de liaison au matériau céramique alumine est mise en œuvre à une température allant de 30 à 120°C, typiquement de 50 à 100°C.

Selon un mode de réalisation, l'étape de liaison de l'aptamère substitué ou de l'anticorps substitué à la molécule de liaison est mise en œuvre en présence d'un ou plusieurs solvants organiques, tel que l'acétonitrile ou une amine par exemple de type triméthylamine ou triéthylamine.

Selon un mode de réalisation, l'étape de liaison de l'aptamère substitué ou de l'anticorps substitué à la molécule de liaison est mise en œuvre en présence d'un catalyseur, supporté ou non supporté, par exemple un catalyseur métallique, par exemple au cuivre, présentant des ligands de type triphénylphosphine. Parmi les catalyseurs qui peuvent être utilisés, on peut citer le bromotris(triphénylphosphine) de cuivre.

Selon un mode de réalisation, l'étape de liaison de l'aptamère substitué ou de l'anticorps substitué à la molécule de liaison est mise en œuvre à une température allant de 30 à 120°C, typiquement de 50 à 100°C, de préférence environ 90°C.

La présente invention a également pour objet un aptamère, un anticorps ou leur combinaison greffé(e) sur un matériau céramique alumine selon l'invention pour la capture de cellules étrangères circulantes, de préférence des cellules tumorales circulantes.

La présente invention a également pour objet un procédé de capture des cellules étrangères circulantes, de préférence des cellules tumorales circulantes, mettant en œuvre le matériau selon l'invention.

### Dispositif

La présente invention a également pour objet un dispositif de capture des cellules étrangères circulantes, de préférence des cellules tumorales circulantes, utilisant le matériau selon l'invention tel que décrit ci-dessus.

Selon un mode de réalisation, le dispositif selon l'invention comprend le matériau selon l'invention tel que décrit ci-dessus comme matériau de capture des cellules tumorales circulantes. Le dispositif peut notamment être similaire dans son fonctionnement à celui décrit dans la demande internationale WO 2017/186626 A1.

De préférence, le dispositif selon l'invention est un dispositif extracorporel, permettant de faire entrer en contact le sang périphérique du patient avec le matériau greffé par au moins un aptamère selon l'invention.

Le dispositif selon l'invention peut comprendre un logement permettant de mettre la circulation sanguine extracorporelle du patient avec le matériau selon l'invention, comprenant au moins une entrée pour l'entrée du sang dans le logement et une sortie pour la sortie du sang du logement. Le logement peut loger un outil de capture.

Dans un mode de réalisation de l'invention, le logement peut présenter une forme tubulaire.

L'outil de capture peut présenter une forme hélicoïdale. En particulier, la structure hélicoïdale ou à vis ou à vis d'Archimède s'étend longitudinalement autour d'un axe de rotation et est fixée à la chambre de capture au moyen d'un système de fixation. De préférence, chaque extrémité de l'axe de rotation est munie d'un aimant. Dans cette variante, le logement comprend en outre un aimant, et notamment de pôle opposé à l'aimant présent sur l'extrémité de l'axe de rotation. Ainsi, la présence des aimants d'une part sur le logement et d'autre part sur les extrémités de l'axe de rotation de l'outil de capture permet de créer un champ magnétique qui maintient en suspension l'outil de capture dans son logement. Ainsi maintenu en lévitation dans le logement du dispositif, l'outil de capture ne se bloque pas par la pression du sang et continue de traiter le sang du patient.

Plus précisément, l'élément de capture mobile a la forme d'une hélice ou d'une spirale. Cette forme particulière est utilisée pour assurer une circulation régulière du fluide sanguin et en même temps une plus grande surface en contact avec le sang circulant afin d'augmenter la probabilité de liaison entre l'entité à enlever, par exemple la cellule tumorale, et les agents liants, par exemple l'anticorps ou l'aptamère. De préférence, la surface hélicoïdale peut s'étendre sur toute la longueur de la chambre de capture. Toutefois, une configuration dans laquelle la longueur de l'hélice est inférieure à la longueur de la chambre de capture et à l'intérieur de laquelle il y a une pluralité de structures hélicoïdales placées en série peut également être utilisée. Bien sûr, grâce à une telle configuration, la longueur de l'ensemble de l'appareil doit nécessairement être augmentée.

Selon les incarnations des inventions présentes, l'élément de capture mobile est configuré pour faciliter le contact entre la surface du réactif de l'appareil et les cellules cibles. Ainsi, tout type d'hélice mobile, double hélice, ou autre structure 3D mobile conçue pour faciliter ce contact est inclus dans cette invention.

Dans un mode de réalisation, l'outil de capture peut être fabriqué ou recouvert en tout ou partie par le matériau selon l'invention. Les parois internes du logement peuvent également être recouvertes en tout ou partie par le matériau selon l'invention.

Le dispositif selon l'invention permet notamment de mettre en contact le volume total de sang d'un patient avec le matériau selon l'invention. En fonction de la taille et du poids du patient, on estime que le volume total de sang d'un patient est compris entre 3L et 5L. De préférence, le dispositif selon l'invention permet de mettre en contact le volume total du sang d'un patient à plusieurs reprises, de sorte à maximiser la capture des cellules tumorales circulantes du patient.

Le dispositif selon l'invention peut également comprendre une pompe afin de pomper le sang du patient. Le dispositif selon l'invention peut également comprendre des filtres.

### FIGURES

La figure 1 présente un schéma de la formule de l'aptamère anti-MUC-1 substitué par un groupe hydrocarboné comprenant un alcyne terminal.

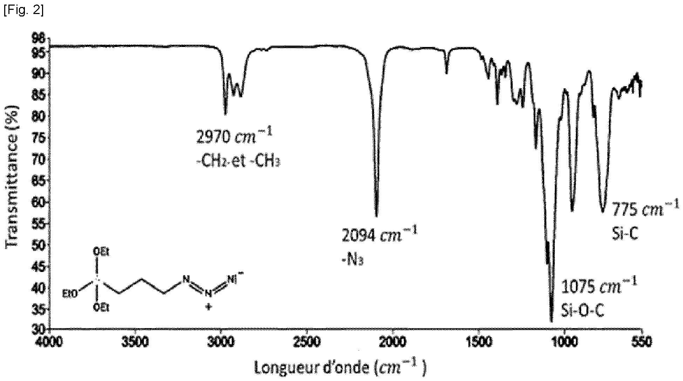

La figure 2 est un spectre infrarouge de l'AzPTES tel que produit avant sa liaison à l'aptamère et la céramique.

La figure 3 est un schéma résumant la réaction sur alumine dense selon l'Exemple 1.

La figure 4 montre des photographies prises en microscopie confocale d'un matériau selon l'invention non greffé (A), d'un matériau selon l'invention greffé par un aptamère marqué au FITC selon l'invention (B) et d'une carte en trois dimensions du matériau selon l'invention greffé par un aptamère marqué au FITC selon l'invention (C).

La figure 5 montre des photographies en microscopie confocale de cellules MDA MB231 en présence ou non d'un aptamère marqué au FITC.

### EXEMPLES

Les exemples suivants sont donnés à titre illustratif de la présente invention, et ne visent en aucun cas à en limiter sa portée.

On décrit ci-dessous des exemples de préparation par greffage d'un aptamère sur une céramique d'alumine afin de préparer une céramique d'alumine greffée par au moins un aptamère conformément à l'invention.

La céramique utilisée est une céramique d'alumine Al₂O₃ dense de type DIN C799, produite par la société SCERAM. Selon les caractéristiques du fournisseur, l'alumine C799 comprend 99.7% d'alumine Al₂O₃ et présente une densité de 3,8 et une densité absolue de 3,9 g/cm3. La porosité ouverte de cette céramique est de 0%.

L'aptamère utilisé est un oligonucléotide d'ADN simple brin de 72 bases conçu par la méthode SELEX pour se lier à la protéine MUC-1 et a pour séquence :

5'GGGAGACAAGAATAAACGCTCAAGCAGTTGATCCTTTGGATACCCTGGTT
CGACAGGAGGCTCACAACAGGC3'

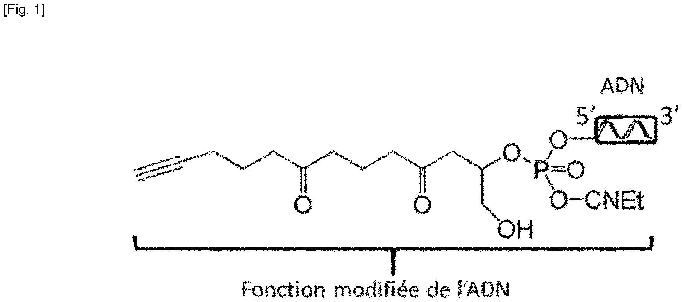

Afin de pouvoir le faire réagir avec d'autres molécules, l'aptamère a été modifié à son extrémité 5' par l'ajout d'un groupe hydrocarboné comprenant un groupe alcyne terminal. La réaction de l'azoture de la molécule de liaison avec le groupe alcyne terminal de l'aptamère substitué forme un cycle triazole. Le schéma de la formule de l'aptamère substitué est représenté à la figure 1. L'aptamère ainsi substitué est utilisé pour la mise en œuvre des Exemples 1 et 2. L'aptamère anti-MUC-1 substitué est produit à façon et obtenu de manière commerciale.

### Exemple 1 Méthode de greffage d'un aptamère anti-MUC-1 sur une céramique d'alumine dense

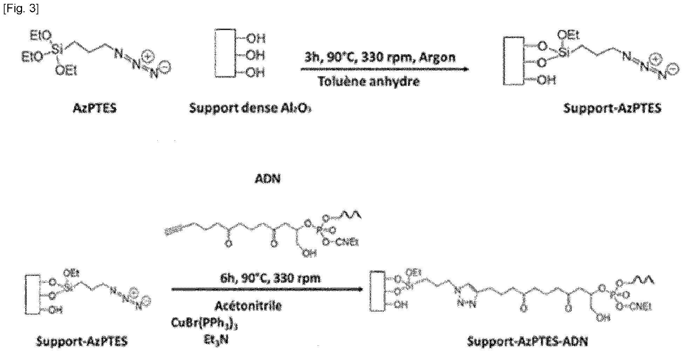

L'exemple 1 illustre la méthode de préparation selon l'invention dans laquelle la molécule de liaison est d'abord liée à la céramique d'alumine avant qu'un aptamère anti-MUC-1 substitué ne soit lié à la molécule de liaison. L'ensemble des réactions de synthèse est résumé à la figure 3.

### Etape 1 - Synthèse de la molécule de liaison 3-azidopropyl)triéthoxysilane (AzPTES) :

On mélange, dans un tube Schlenk de 50 ml, 1,93 g de chlorosilane (8 mmol), 0.2g de KI (1.2 mmol) et 1.56g de NaN₃ (24 mmol). Le système est mis sous vide puis sous atmosphère d'argon et 10 mL de DMF (N,N-dimethylformamide) sont introduits sous argon. La réaction est lancée pendant 24 h à 80 °C et 330 rpm. La solution est alors jaune. Au bout de 24 h, la solution est devenue blanche et présente deux phases. Une fois le milieu revenu à température ambiante, la solution est filtrée sur un filtre Célite® 545 (environ 1 cm) imbibée de 15 ml de DMF sur un fritté n°3, puis rincée avec 5 ml de DMF. Le DMF est évaporé à l'aide d'un évaporateur rotatif (70°C, 90 rpm).

Le dichlorométhane (30 ml) est ajouté à la solution. Celle-ci est lavée deux fois avec 30 mL d'eau, puis la phase organique est séchée sur MgSO₄, puis filtrée et placée à l'évaporateur rotatif (40°C, 90 rpm) afin d'éliminer le dichlorométhane et les éventuelles traces de DMF. Des résidus blancs sont observés. La solution est centrifugée pendant 5 mn à 1500 rpm. Le surnageant jaune d'aspect huileux, contenant l'AzPTES est récupéré puis pesé : 1,1 g (4 mmol) de produit sont récupéré. Le rendement molaire de cette étape est d'environ 56 %. L'AzPTES a pour formule :

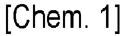

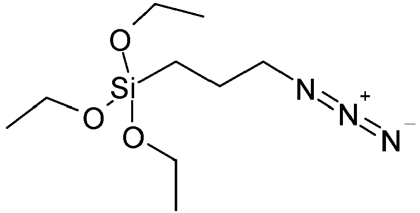

Les résultats de caractérisation de l'AzPTES aux infrarouges et par RMN sont les suivants :
- FR-IR (ATR, résolution 4 cm⁻¹, 8 analyses/échantillon): Bande observée à 2100 cm⁻¹ caractéristique du groupement N3. Autres bandes observées : 1075 cm-1 caractéristique du groupement Si-O-C, 775 cm⁻¹ caractéristique du groupement Si-C. Le spectre infrarouge de l'AzPTES est présenté à la figure 3.
- RMN 1H (500 MHz, CDCl3, 25°C, δ, ppm) : 0.69 (m, 2H, CH2-Si), 1.23 (t, 9H, CH3, J = 7.0 Hz), 1.71 (m, 2H, CH2), 3.26 (t, 2H, CH2-N3, J = 6.9 Hz), 3.83 (q, 6H, CH2-O, J = 7.0 Hz)
- RMN 13C (500 MHz, CDCl3, 25°C, δ, ppm) : 7.7 (CH2-Si), 18.3 (CH3), 22.7 (CH2), 53.9 (CH2-N3), 58,5 (CH2-O)

### Etape 2 - Liaison de l'AzPTES à la céramique d'alumine.

On utilise une pastille de céramique d'alumine de 14 mm de diamètre 14 mm, et 4 mm d'épaisseur.

La pastille de céramique d'alumine Al₂O₃ est mis à l'étuve à 120°C pendant une nuit. Un ballon bicol de 50 mL est placé 5 mn à l'étuve à 120°C afin d'enlever toute trace d'humidité dans le ballon. La céramique est ensuite introduite dans le bicol. Celui-ci est mis sous vide puis sous argon. 7 ml de toluène anhydre est introduit sous argon. Le mélange est porté à 60°C, 330 rpm. Une fois la température de 60°C atteinte, 2 nmol d'AzPTES est introduit sous argon, goutte à goutte, puis un réfrigérant est installé sur le bicol et mis sous argon. La réaction est réalisée pendant 3 h à 90°C, 330 rpm.

Au bout de 3 h, la céramique est lavée par 10 ml de toluène pendant 2min dans un bain à ultrasons (eau, puissance : 100 %, 80 Hz) trois fois, puis une fois avec 10 ml d'éthanol 95%.

Une analyse par spectrométrie photoéletronique X (XPS) détecte la présence de silicium et d'azote, preuve que la liaison de l'AzPTES lié à l'ADN à la céramique alumine a fonctionné. En effet, le silicium et l'azote ne font pas partie des éléments chimiques présents dans la céramique. La présence de silicium à la surface du matériau indique que la liaison de l'AzPTES au matériau a fonctionné et la présence d'azote indique la présence de l'ADN simple brin formant l'aptamère.

### Etape 3 - Liaison de l'aptamère anti-MUC-1 à l'AzPTES lié à la céramique d'alumine.

On mélange 4.8 mg de catalyseur CuBr(PPh₃)₃, 1.5 µl de triméthylamine et 7 ml d'acétonitrile avec la céramique lié à l'AzPTES. L'ensemble est porté à 60°C, 330 rpm. Une fois la température de 60°C atteinte, l'ADN est introduit goutte à goutte. Un réfrigérant est placé sur le ballon et la réaction est réalisée pendant 6h à 90°C, 330 rpm pour liaison de l'aptamère à l'AzPTES. Le milieu réactionnel est alors transparent.

À la fin de la réaction, le catalyseur (CuBr(PPh₃)₃) est éliminé par filtration de la solution grâce à un filtre (porosité 0,45 µm) sur seringue. La céramique est lavée trois fois par de l'acétonitrile puis une fois à l'éthanol dans un bain à ultrasons comme décrit à l'étape 1.

La céramique d'alumine est ensuite nettoyée avec 3 mL d'éthanol à 95% stérile. La pastille est ensuite stockée à 4°C pour éviter ou réduire la dégradation de l'aptamère greffé sur la céramique. Le matériau ainsi greffé peut être stocké pendant 1 mois à 4°C.

Le milieu réactionnel et les milieux de lavages (acétonitrile et éthanol) sont additionnés puis placés à l'évaporateur rotatif à 40°C, 90 rpm. Une fois les solvants évaporés, 200 µL d'eau ultrapure sont ajoutés à l'ADN désorbé par les lavages. L'ADN non greffé est alors quantifié en UV visible à 260 nm et 280 nm selon toute méthode bien connue de l'homme du métier.

Une analyse infrarouge (ATR, résolution 4 cm⁻¹, 8 analyses/échantillon) permet d'observer une bande à 3325,35 cm⁻¹, caractéristique des fonctions -NH de l'ADN, ainsi qu'une bande large à 2114,90 cm⁻¹ caractéristique du groupement alcyne, et une bande à 1639,12 cm⁻¹ caractéristique des cétones de l'ADN.

Le rendement de liaison de l'aptamère sur le matériau céramique selon la méthode de l'exemple 2 est d'environ 44%.

### Exemple 2

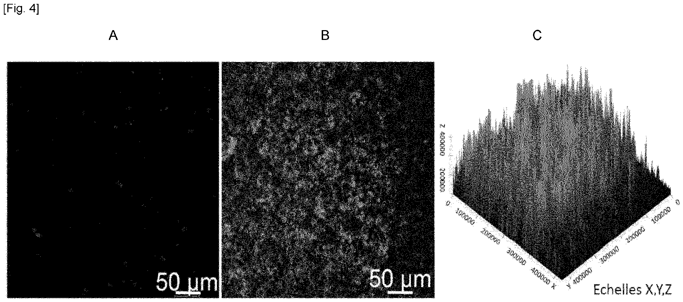

L'exemple 1 a été réitéré en utilisant un aptamère marqué à son extrémité 3' par le FITC. Le matériau ainsi préparé a été observé au microscope confocal. Un matériau en céramique d'alumine non marqué a également été observé comme témoin : comme on peut le voir sur la figure 4A, aucune fluorescence n'est visible à la surface du matériau.

Il a été observé que le matériau préparé conformément au procédé selon l'invention était greffé de manière homogène par l'aptamère (figure 4B). Une carte en trois dimensions a été réalisée en balayant le matériau greffé de la figure 4B par un laser sur une épaisseur de 8 µm avec un pas de 0.8 µm, ce qui correspond à la prise d'une image confocale tous les 0.8 µm. La compilation numérique de ces images permet d'obtenir la carte en trois dimensions présentées à la figure 4C. Comme le montre cette carte, le matériau selon l'invention est greffé par l'aptamère marqué au FITC de manière dense et homogène (figure 4C).

### Exemple 3

Des essais de fixation *in vitro* ont été menés sur des cellules de cancer du sein MDA MB231. Les cellules MDA MB231 sont des cellules épithéliales issues d'un adénocarcinome du sein. Les cellules MDA MB231 sont mises en culture conformément aux instructions du fournisseur. Les cellules sont ensuite trypsinées, lavées et centrifugées 10min à 1500 rpm. Pour le test, 20 000 cellules sont reprises dans 500µl de milieu de culture cellulaire sans sérum de veau fœtal (SVF). Trois conditions sont testées :
- Témoin négatif: milieu de culture (20 µl),
- FITC seul dilué dans le milieu de culture (20 µl), ou
- Aptamère marqué au FITC dans du milieu de culture (20 µl).
L'aptamère utilisé est un aptamère spécifique du récepteur MUC1 et comprend 72 bases comme suit :

5'-GGGAGACAAGAATAAACGCTCAAGCAGTTGATCCTTTGGATACCCTG
GTTCGACAGGAGGCTCACAACAGGC-3'

L'aptamère est marqué par l'isothiocyanate de fluorescéine FITC à son extrémité 3' selon toute technique bien connue de l'homme du métier.

Les cellules sont ensuite incubées 1h à 37°C et 5% de CO₂ avant d'être transférées dans une plaque 24 puits et incubées 12h-16h à 37°C et 5% de CO₂. Les cellules sont ensuite lavées et fixées avec 500µl de PBS PAF à 4% avant d'être observées en microscopie confocale, avec ou sans fluorescence, avec un grossissement x10.

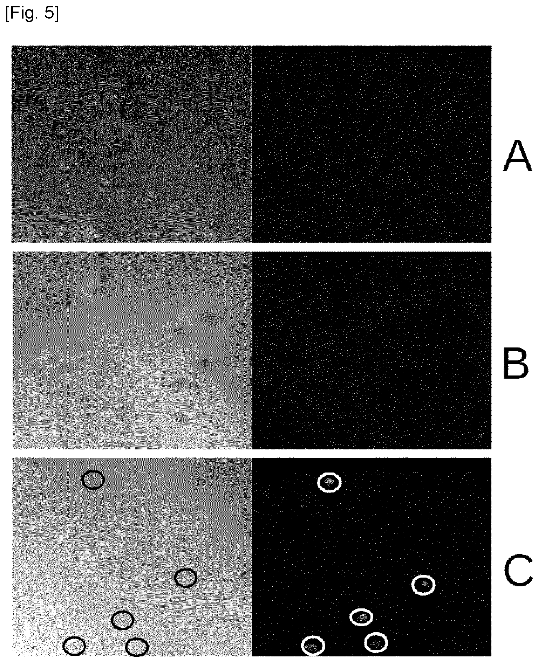

La figure 5A montre des photographies des cellules cultivées en puits dans la condition témoin négatif, c'est-à-dire les cellules cultivées dans du milieu de culture sans marqueur de fluorescence. La photographie de gauche correspond à l'observation sans fluorescence et la photographie de droite montre les cellules observées avec fluorescence. Aucune fluorescence n'est observée sur les cellules. Les cellules n'émettent donc aucune fluorescence spontanée.

La figure 5B montre des photographies des cellules cultivées en puits dans la condition témoin FITC seul, c'est-à-dire les cellules cultivées dans du milieu de culture contenant du FITC mais pas d'aptamère. La photographie de gauche correspond à l'observation sans fluorescence et la photographie de droite montre les cellules observées avec fluorescence. Aucune fluorescence n'est observée sur les cellules. Le fluorochrome FITC ne se fixe donc pas spontanément sur les cellules.

La figure 5C montre des photographies des cellules cultivées en puits avec l'aptamère marqué au FITC. La photographie de gauche correspond à l'observation sans fluorescence et la photographie de droite montre les cellules observées avec fluorescence. Comme on peut le voir, les cellules sur lesquelles l'aptamère marqué au FITC s'est fixé émettent une fluorescence.

### Conclusion

La présente invention fournit donc un matériau céramique alumine greffé par un aptamère, un anticorps ou leur combinaison, le greffage se faisant de manière covalente, de préférence par l'intermédiaire d'une molécule de liaison, ainsi qu'un procédé de préparation d'un tel matériau. Le matériau selon l'invention permet donc de proposer un matériau biocompatible et hémocompatible pour la capture de cellules étrangères, de préférence des cellules tumorales circulantes présentes dans le sang d'un patient. Le matériau selon l'invention est inerte vis-à-vis du sang du patient de sorte qu'il ne capture aucun autre élément circulant dans le sang du patient que les cellules étrangères ciblées et qu'il ne provoque aucune déplétion, dénaturation, coagulation ou réaction susceptible de nuire au patient. Le matériau selon l'invention permet en outre de traiter le volume total sanguin d'un patient, voire de permettre plusieurs passages du volume sanguin total au contact du matériau pour capturer et retirer le maximum possible de cellules circulantes du sang du patient. Le matériau selon l'invention peut donc être utilisé à des fins thérapeutiques pour réduire le risque de formation de métastases ou le risque de récidive d'un cancer. Le matériau selon l'invention peut également être utilisé pour améliorer l'efficacité d'un traitement chimiothérapeutique.

Le matériau selon l'invention peut également être utilisé dans le cadre d'un dispositif de capture de cellules étrangères circulantes, de préférence un dispositif extracorporel de capture des cellules tumorales circulantes. En effet, contrairement aux dispositifs de l'art antérieur dans lesquels les aptamères et/ou anticorps sont adsorbés à la surface du matériau, le matériau selon l'invention est greffé de manière covalente par l'au moins un aptamère et/ou au moins un anticorps. Ce greffage permet d'éviter qu'un aptamère et/ou un anticorps ne se détache, entrainé par le flux, et ne passe dans la circulation sanguine du patient.

## Revendications

1. Matériau céramique alumine greffé par au moins un aptamère, au moins un anticorps ou leurs combinaisons.

2. Matériau selon la revendication 1 dans lequel l'au moins un aptamère, l'au moins un anticorps ou leur combinaison est greffé audit matériau céramique alumine par une molécule de liaison.

3. Matériau selon la revendication 2 dans lequel la molécule de liaison est un trialkoxysilane de formule ⁻N=N⁺=N-L-Si(OR1)(OR2)(OR3) dans laquelle L désigne un radical hydrocarboné divalent linéaire, ramifié ou cyclique comprenant 1 à 20 atomes de carbone, de préférence 1 à 10 atomes de carbone, et R1, R2, R3 sont identiques ou différents et représentent un groupe alkyle linéaire ou ramifié comprenant 1 à 6 atomes de carbone, de préférence 1 à 3 atomes de carbone.

4. Matériau selon l'une des revendications précédentes dans lequel l'au moins un aptamère est de l'ADN simple brin.

5. Matériau selon l'une des revendications précédentes dans lequel l'au moins un aptamère ou l'au moins un anticorps est un aptamère substitué ou un anticorps substitué par au moins un groupe hydrocarboné linéaire, ramifié ou cyclique comprenant une fonction alcyne terminal ayant pour formule C=CH et de 3 à 20 atomes de carbone, de préférence de 10 à 15 atomes de carbone, ledit groupe hydrocarboné étant éventuellement substitué par un ou plusieurs hétéroatomes.

6. Matériau selon l'une des revendications précédentes présentant au moins une surface en contact avec le sang d'un patient, ladite surface n'étant pas poreuse.

7. Matériau selon l'une des revendications précédentes dans lequel l'au moins un aptamère ou l'au moins un anticorps est capable de se lier à au moins l'une des protéines choisies parmi mucine 1 (MUC-1), EpCam, HER-2, EGFR, vimentine, E-cadhérine, N-cadhérine, l'OB-cadhérine, le CD44v6, le CD44v8, PSA, cytokératine 8, cytokératine 19, ABC-G2 et leurs combinaisons, de préférence la protéine mucine 1 (MUC-1).

8. Matériau selon l'une des revendications précédentes dans lequel l'au moins un aptamère, l'au moins un anticorps ou leur combinaison est lié à au moins une molécule de marquage.

9. Aptamère, anticorps ou leur combinaison greffé sur le matériau céramique alumine selon l'une des revendications 1 à 8 pour la capture de cellules étrangères circulantes, de préférence de cellules tumorales circulantes.

10. Aptamère, anticorps ou leur combinaison pour son utilisation selon la revendication 9 dans laquelle les cellules tumorales circulantes sont issues d'un cancer du sein, d'un cancer du poumon, d'un cancer de la prostate, d'un cancer colorectal, d'un cancer de la vessie, d'un cancer de l'estomac, d'un mélanome.

11. Procédé de préparation du matériau selon l'une des revendications 1 à 8, comprenant le greffage de la céramique alumine par au moins un aptamère, au moins un anticorps ou leur combinaison.

12. Procédé selon la revendication 11, dans lequel l'au moins un aptamère, au moins un anticorps ou leur combinaison est greffé audit matériau céramique alumine par une molécule de liaison, ledit procédé comprenant au moins les étapes suivantes :
- liaison de la molécule de liaison au matériau céramique alumine par réaction d'une ou plusieurs fonctions alkoxy de la molécule de liaison avec un ou plusieurs groupements hydroxyles du matériau céramique alumine ; et
- liaison de l'aptamère ou de l'anticorps à la molécule de liaison par réaction de chimie click.

13. Procédé selon la revendication 11 ou 12 dans lequel l'au moins un aptamère ou l'au moins un anticorps est un aptamère substitué ou un anticorps substitué par au moins un groupe hydrocarboné linéaire, ramifié ou cyclique comprenant une fonction alcyne terminal ayant pour formule C=CH et de 3 à 20 atomes de carbone, de préférence de 10 à 15 atomes de carbone, ledit groupe hydrocarboné étant éventuellement substitué par un ou plusieurs hétéroatomes.

14. Procédé selon l'une des revendications 10 à 13 dans lequel l'aptamère ou l'anticorps est substitué et la liaison de l'aptamère substitué ou de l'anticorps substitué à la molécule de liaison est mise en œuvre par réaction de la fonction alcyne terminale de l'aptamère substitué ou de l'anticorps substitué avec la fonction azoture terminale de formule -N=N⁺=N⁻ de la molécule de liaison.

15. Dispositif de capture des cellules étrangères circulantes, de préférence des cellules tumorales circulantes, comprenant le matériau selon l'une des revendications 1 à 8 et au moins un logement.
